# EUROPEAN PATENT SPECIFICATION

(11) **EP 0 690 721 B1**
(45) Date of publication and mention of the grant of the patent: **13.05.1998**
(21) Application number: 94910876.5
(22) Date of filing: 11.03.1994
(51) Int. Cl.: A61K 38/00

(54) **TREATMENT OF MYCOBACTERIAL DISEASES BY ADMINISTRATION OF BACTERICIDAL/PERMEABILITY-INCREASING PROTEIN PRODUCTS**
BEHANDLUNG VON MYCOBAKTERIELLEN ERKRANKUNGEN DURCH VERABREICHUNG VON BAKRERIENTÖTENDE, DURCHLÄSSIGKEITSERHÖHENDE PROTEINFRAGMENTE
TRAITEMENT DE MALADIES MYCOBACTERIENNES PAR ADMINSTRATION DE PRODUITS PROTEIQUES BACTERICIDES/AUGMENTANT LA PERMEABILITE

(30) Priority: 12.03.1993 US 31145
(43) Date of publication of application: 10.01.1996
(73) Proprietor: Xoma Corporation, Berkeley California 94710 (US)
(72) Inventor: LAMBERT, Lewis H., Jr., Fremont, CA 94539 (US)
(74) Representative: Brown, John David
(86) International application number: US9402463
(87) International publication number: WO9420129

(56) References cited:
- WO-A-92/03535
- WO-A-92/09621
- SURGERY vol. 114, no. 2 , August 1993 , ST LOUIS pages 140 - 146 KELLY C.J. ET AL. 'Role of bactericidal permeability-increasing protein in the treatment of gram-negative pneumonia'

SEQUENCE LISTING

(1) GENERAL INFORMATION:

(i) APPLICANT: XOMA Corporation

(ii) TITLE OF INVENTION: Treatment of Mycobacterial Diseases by
Administration of Bactericidal/Permeability-Increasing Protein Product

(iii) NUMBER OF SEQUENCES: 2

(iv) CORRESPONDENCE ADDRESS:
(A) ADDRESSEE: Marshall, O'Toole, Gerstein, Murray & Borun
(B) STREET: 6300 Sears Tower, 233 South Wacker Drive
(C) CITY: Chicago
(D) STATE: Illinois
(E) COUNTRY: USA
(F) ZIP: 60606-6402

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(viii) ATTORNEY INFORMATION:
(A) NAME: Borun, Michael F.
(B) REGISTRATION NUMBER: 25,447
(C) REFERENCE/DOCKET NUMBER: 31293

(ix) TELECOMMUNICATION INFORMATION:
(A) TELEPHONE: 312/474-6300
(B) TELEFAX: 312/474-0448
(C) TELEX: 25-3856

(2) INFORMATION FOR SEQ ID NO:1:

(i) SEQUENCE CHARACTERISTICS:
(A) LENGTH: 1813 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 31..1491

(ix) FEATURE:
(A) NAME/KEY: mat_peptide
(B) LOCATION: 124..1491

(xi) SEQUENCE DESCRIPTION: SEQ ID NO:1:

CAGGCCTTGA GGTTTTGGCA GCTCTGGAGG ATG AGA GAG AAC ATG GCC AGG GGC 54
Met Arg Glu Asn Met Ala Arg Gly
-31 -30 -25

CCT TGC AAC GCG CCG AGA TGG GTG TCC CTG ATG GTG CTC GTC GCC ATA 102
Pro Cys Asn Ala Pro Arg Trp Val Ser Leu Met Val Leu Val Ala Ile
-20 -15 -10

GGC ACC GCC GTG ACA GCG GCC GTC AAC CCT GGC GTC GTG GTC AGG ATC 150
Gly Thr Ala Val Thr Ala Ala Val Asn Pro Gly Val Val Val Arg Ile
-5 1 5

TCC CAG AAG GGC CTG GAC TAC GCC AGC CAG CAG GGG ACG GCC GCT CTG 198
Ser Gln Lys Gly Leu Asp Tyr Ala Ser Gln Gln Gly Thr Ala Ala Leu
10 15 20 25

CAG AAG GAG CTG AAG AGG ATC AAG ATT CCT GAC TAC TCA GAC AGC TTT 246
Gln Lys Glu Leu Lys Arg Ile Lys Ile Pro Asp Tyr Ser Asp Ser Phe
30 35 40

AAG ATC AAG CAT CTT GGG AAG GGG CAT TAT AGC TTC TAC AGC ATG GAC 294
Lys Ile Lys His Leu Gly Lys Gly His Tyr Ser Phe Tyr Ser Met Asp
45 50 55

ATC CGT GAA TTC CAG CTT CCC AGT TCC CAG ATA AGC ATG GTG CCC AAT 342
Ile Arg Glu Phe Gln Leu Pro Ser Ser Gln Ile Ser Met Val Pro Asn
60 65 70

GTG GGC CTT AAG TTC TCC ATC AGC AAC GCC AAT ATC AAG ATC AGC GGG 390
Val Gly Leu Lys Phe Ser Ile Ser Asn Ala Asn Ile Lys Ile Ser Gly
75 80 85

AAA TGG AAG GCA CAA AAG AGA TTC TTA AAA ATG AGC GGC AAT TTT GAC 438
Lys Trp Lys Ala Gln Lys Arg Phe Leu Lys Met Ser Gly Asn Phe Asp
90 95 100 105

CTG AGC ATA GAA GGC ATG TCC ATT TCG GCT GAT CTG AAG CTG GGC AGT 486
Leu Ser Ile Glu Gly Met Ser Ile Ser Ala Asp Leu Lys Leu Gly Ser
110 115 120

AAC CCC ACG TCA GGC AAG CCC ACC ATC ACC TGC TCC AGC TGC AGC AGC 534
Asn Pro Thr Ser Gly Lys Pro Thr Ile Thr Cys Ser Ser Cys Ser Ser
125 130 135

CAC ATC AAC AGT GTC CAC GTG CAC ATC TCA AAG AGC AAA GTC GGG TGG 582
His Ile Asn Ser Val His Val His Ile Ser Lys Ser Lys Val Gly Trp
140 145 150

CTG ATC CAA CTC TTC CAC AAA AAA ATT GAG TCT GCG CTT CGA AAC AAG 630
Leu Ile Gln Leu Phe His Lys Lys Ile Glu Ser Ala Leu Arg Asn Lys
155 160 165

ATG AAC AGC CAG GTC TGC GAG AAA GTG ACC AAT TCT GTA TCC TCC AAG 678
Met Asn Ser Gln Val Cys Glu Lys Val Thr Asn Ser Val Ser Ser Lys
170 175 180 185

CTG CAA CCT TAT TTC CAG ACT CTG CCA GTA ATG ACC AAA ATA GAT TCT 726
Leu Gln Pro Tyr Phe Gln Thr Leu Pro Val Met Thr Lys Ile Asp Ser
190 195 200

GTG GCT GGA ATC AAC TAT GGT CTG GTG GCA CCT CCA GCA ACC ACG GCT 774
Val Ala Gly Ile Asn Tyr Gly Leu Val Ala Pro Pro Ala Thr Thr Ala
205 210 215

GAG ACC CTG GAT GTA CAG ATG AAG GGG GAG TTT TAC AGT GAG AAC CAC 822
Glu Thr Leu Asp Val Gln Met Lys Gly Glu Phe Tyr Ser Glu Asn His
220 225 230

CAC AAT CCA CCT CCC TTT GCT CCA CCA GTG ATG GAG TTT CCC GCT GCC 870
His Asn Pro Pro Pro Phe Ala Pro Pro Val Met Glu Phe Pro Ala Ala
235 240 245

CAT GAC CGC ATG GTA TAC CTG GGC CTC TCA GAC TAC TTC TTC AAC ACA 918
His Asp Arg Met Val Tyr Leu Gly Leu Ser Asp Tyr Phe Phe Asn Thr
250 255 260 265

GCC GGG CTT GTA TAC CAA GAG GCT GGG GTC TTG AAG ATG ACC CTT AGA 966
Ala Gly Leu Val Tyr Gln Glu Ala Gly Val Leu Lys Met Thr Leu Arg
270 275 280

GAT GAC ATG ATT CCA AAG GAG TCC AAA TTT CGA CTG ACA ACC AAG TTC 1014
Asp Asp Met Ile Pro Lys Glu Ser Lys Phe Arg Leu Thr Thr Lys Phe
285 290 295

TTT GGA ACC TTC CTA CCT GAG GTG GCC AAG AAG TTT CCC AAC ATG AAG 1062
Phe Gly Thr Phe Leu Pro Glu Val Ala Lys Lys Phe Pro Asn Met Lys
300 305 310

ATA CAG ATC CAT GTC TCA GCC TCC ACC CCG CCA CAC CTG TCT GTG CAG 1110
Ile Gln Ile His Val Ser Ala Ser Thr Pro Pro His Leu Ser Val Gln
315 320 325

CCC ACC GGC CTT ACC TTC TAC CCT GCC GTG GAT GTC CAG GCC TTT GCC 1158
Pro Thr Gly Leu Thr Phe Tyr Pro Ala Val Asp Val Gln Ala Phe Ala
330 335 340 345

GTC CTC CCC AAC TCC TCC CTG GCT TCC CTC TTC CTG ATT GGC ATG CAC 1206
Val Leu Pro Asn Ser Ser Leu Ala Ser Leu Phe Leu Ile Gly Met His
350 355 360

ACA ACT GGT TCC ATG GAG GTC AGC GCC GAG TCC AAC AGG CTT GTT GGA 1254
Thr Thr Gly Ser Met Glu Val Ser Ala Glu Ser Asn Arg Leu Val Gly
365 370 375

GAG CTC AAG CTG GAT AGG CTG CTC CTG GAA CTG AAG CAC TCA AAT ATT 1302
Glu Leu Lys Leu Asp Arg Leu Leu Leu Glu Leu Lys His Ser Asn Ile
380 385 390

GGC CCC TTC CCG GTT GAA TTG CTG CAG GAT ATC ATG AAC TAC ATT GTA 1350
Gly Pro Phe Pro Val Glu Leu Leu Gln Asp Ile Met Asn Tyr Ile Val
395 400 405

CCC ATT CTT GTG CTG CCC AGG GTT AAC GAG AAA CTA CAG AAA GGC TTC 1398
Pro Ile Leu Val Leu Pro Arg Val Asn Glu Lys Leu Gln Lys Gly Phe
410 415 420 425

CCT CTC CCG ACG CCG GCC AGA GTC CAG CTC TAC AAC GTA GTG CTT CAG 1446
Pro Leu Pro Thr Pro Ala Arg Val Gln Leu Tyr Asn Val Val Leu Gln
430 435 440

CCT CAC CAG AAC TTC CTG CTG TTC GGT GCA GAC GTT GTC TAT AAA 1491
Pro His Gln Asn Phe Leu Leu Phe Gly Ala Asp Val Val Tyr Lys
445 450 455

TGAAGGCACC AGGGGTGCCG GGGGCTGTCA GCCGCACCTG TTCCTGATGG GCTGTGGGGC 1551

ACCGGCTGCC TTTCCCCAGG GAATCCTCTC CAGATCTTAA CCAAGAGCCC CTTGCAAACT 1611

TCTTCGACTC AGATTCAGAA ATGATCTAAA CACGAGGAAA CATTATTCAT TGGAAAAGTG 1671

CATGGTGTGT ATTTTAGGGA TTATGAGCTT CTTTCAAGGG CTAAGGCTGC AGAGATATTT 1731

CCTCCAGGAA TCGTGTTTCA ATTGTAACCA AGAAATTTCC ATTTGTGCTT CATGAAAAAA 1791

AACTTCTGGT TTTTTTCATG TG 1813

(2) INFORMATION FOR SEQ ID NO:2:

(i) SEQUENCE CHARACTERISTICS:
(A) LENGTH: 487 amino acids
(B) TYPE: amino acid
(D) TOPOLOGY: linear

(ii) MOLECULE TYPE: protein

(xi) SEQUENCE DESCRIPTION: SEQ ID NO:2:

Met Arg Glu Asn Met Ala Arg Gly Pro Cys Asn Ala Pro Arg Trp Val
-31 -30 -25 -20

Ser Leu Met Val Leu Val Ala Ile Gly Thr Ala Val Thr Ala Ala Val
-15                 -10                 -5                  1

Asn Pro Gly Val Val Val Arg Ile Ser Gln Lys Gly Leu Asp Tyr Ala
            5                   10                  15

Ser Gln Gln Gly Thr Ala Ala Leu Gln Lys Glu Leu Lys Arg Ile Lys
        20                  25                  30

Ile Pro Asp Tyr Ser Asp Ser Phe Lys Ile Lys His Leu Gly Lys Gly
    35                  40                  45

His Tyr Ser Phe Tyr Ser Met Asp Ile Arg Glu Phe Gln Leu Pro Ser
50                  55                  60                  65

Ser Gln Ile Ser Met Val Pro Asn Val Gly Leu Lys Phe Ser Ile Ser
                70                  75                  80

Asn Ala Asn Ile Lys Ile Ser Gly Lys Trp Lys Ala Gln Lys Arg Phe
            85                  90                  95

Leu Lys Met Ser Gly Asn Phe Asp Leu Ser Ile Glu Gly Met Ser Ile
        100                 105                 110

Ser Ala Asp Leu Lys Leu Gly Ser Asn Pro Thr Ser Gly Lys Pro Thr
    115                 120                 125

Ile Thr Cys Ser Ser Cys Ser Ser His Ile Asn Ser Val His Val His
130                 135                 140                 145

Ile Ser Lys Ser Lys Val Gly Trp Leu Ile Gln Leu Phe His Lys Lys
                150                 155                 160

Ile Glu Ser Ala Leu Arg Asn Lys Met Asn Ser Gln Val Cys Glu Lys
            165                 170                 175

Val Thr Asn Ser Val Ser Ser Lys Leu Gln Pro Tyr Phe Gln Thr Leu
        180                 185                 190

Pro Val Met Thr Lys Ile Asp Ser Val Ala Gly Ile Asn Tyr Gly Leu
    195                 200                 205

Val Ala Pro Pro Ala Thr Thr Ala Glu Thr Leu Asp Val Gln Met Lys
210                 215                 220                 225

Gly Glu Phe Tyr Ser Glu Asn His His Asn Pro Pro Pro Phe Ala Pro
                230                 235                 240

Pro Val Met Glu Phe Pro Ala Ala His Asp Arg Met Val Tyr Leu Gly
            245                 250                 255

Leu Ser Asp Tyr Phe Phe Asn Thr Ala Gly Leu Val Tyr Gln Glu Ala
        260                 265                 270

Gly Val Leu Lys Met Thr Leu Arg Asp Asp Met Ile Pro Lys Glu Ser
    275                 280                 285

Lys Phe Arg Leu Thr Thr Lys Phe Phe Gly Thr Phe Leu Pro Glu Val
290                 295                 300                 305

Ala Lys Lys Phe Pro Asn Met Lys Ile Gln Ile His Val Ser Ala Ser
                310                 315                 320

Thr Pro Pro His Leu Ser Val Gln Pro Thr Gly Leu Thr Phe Tyr Pro
                325                 330                 335

Ala Val Asp Val Gln Ala Phe Ala Val Leu Pro Asn Ser Ser Leu Ala
            340                 345                 350

Ser Leu Phe Leu Ile Gly Met His Thr Thr Gly Ser Met Glu Val Ser
        355                 360                 365

Ala Glu Ser Asn Arg Leu Val Gly Glu Leu Lys Leu Asp Arg Leu Leu
370 375 380 385

Leu Glu Leu Lys His Ser Asn Ile Gly Pro Phe Pro Val Glu Leu Leu
390 395 400

Gln Asp Ile Met Asn Tyr Ile Val Pro Ile Leu Val Leu Pro Arg Val
                405                 410                 415

Asn Glu Lys Leu Gln Lys Gly Phe Pro Leu Pro Thr Pro Ala Arg Val
            420                 425                 430

Gln Leu Tyr Asn Val Val Leu Gln Pro His Gln Asn Phe Leu Leu Phe
        435                 440                 445

Gly Ala Asp Val Val Tyr Lys
    450                 455

## Description

### Background of the Invention

The present invention relates to treatment of a subject suffering from infection with *Mycobacteria* by administration of Bactericidal/Permeability-Increasing Protein (BPI) protein products and provides the use of a BPI protein product for the manufacture of a medicament for the treatment of Mycobacteria infection. *Mycobacterium* is a non-motile, acid-fast, aerobic, Gram-positive genus of bacteria known to cause grave human and animal diseases, such as tuberculosis and leprosy. Infections caused by *M.avium* are the most common form of disseminated bacterial disease in AIDS patients. Orme, *et al., Infect. and Immun., 61(1)*:338-342 (1993).

The administration of conventional antibiotics to treat *Mycobacterial* infection is known in the art and has achieved varying success depending on the susceptibility of the bacterial strain, the efficacy and toxicity of the antibiotic(s) employed, the duration of treatment, and numerous other factors. Antimicrobials that have been employed alone or in combination in the United States to treat tuberculosis include isoniazid, rifampin, ethambutol, *p*-aminosalicylic acid, pyrazinamide, streptomycin, capreomycin, cycloserine, ethionamide, and kanamycin. McClatchy, *Antimycobacterial Drugs: Mechanisms of Action, Drug Resistance, Susceptibility Testing, and Assays of Activity in Biological Fluids*, pp. 134-197, *In* Antibiotics in Laboratory Medicine, 3rd ed., V. Lorian, ed. The Williams & Wilkins Co., Baltimore (1991). As many *Mycobacterial* strains are drug resistant, serious obstacles exist for control and successful treatment of tuberculosis and other *Mycobacterial* diseases. *Id*.

A variety of factors have made treatment of individuals afflicted with *Mycobacterial* diseases problematic. First, *Mycobacteria* possess a very hydrophobic cell wall that affords protection against the host's immune system. As *Mycobacterial* infections tend to be chronic, the pathologies of these organisms are generally due to host response. Also, many *Mycobacterial* strains are drug-resistant. These and other factors make the development of novel, effective methods for treating *Mycobacterial* diseases highly desirable.

*Mycobacteria* are readily distinguished from Gram-negative bacteria by selective staining (i.e., Acid-fast or Gram's stain) due to significant differences in cell wall structure. Gram-negative bacteria are characterized by a cell wall composed of a thin layer of peptidoglycan covered by an outer membrane of lipoprotein and lipopolysaccharide (LPS), whereas Gram-positive bacteria have a cell wall with a thicker layer of peptidoglycan with attached teichoic acids, but no LPS. One of the major constituents widely distributed within the cell wall of *Mycobacterium* species is lipoarabinomannan (LAM). LAM has been purified from both *M. leprae* and *M. tuberculosis*. Hunter *et al., J Biol. Chem., 261*:12345-12351 (1986). LAM is a serologically active mannose containing phosphorylated lipopolysaccharide that may be membrane associated.

The complex physiological effects of LAM appear to be concentration, time, and source-dependent. For example, Chaterjee *et al*., *Infect. and Immun., 60(3)*:1249-1253 (1992), reported that, in the first 24 hours following exposure, LAM from an avirulent strain of tuberculosis was 100-fold more potent at stimulating TNF secretion in mouse macrophages than LAM from a virulent strain. LAM concentrations of 0.01-10 µg/ml for the avirulent strain and 0.01-100 µg/ml for the virulent strain were tested, and increased LAM concentration was associated with increased TNF production with LAM from both species.

Macrophage-inhibitory effects of LAM have also been described in the art. LAM purified from both *M. leprae* and *M. tuberculosis* has been reported to be a potent *in vitro* inhibitor of T-cell lymphokine activation of mouse macrophages. Sibley *et al., Infection and Immunity, 56(5)*:1232-1236 (1988). Because the principle efferent role of the macrophage in acquired resistance to intracellular pathogens requires activation by T-cell lymphokines, notably gamma-interferon (IFN-γ), macrophages whose activation-response is inhibited are severely compromised in their capacity for both enhanced microbicidal and tumoricidal activities.

In another study, Sibley *et al., Clin. Exp. Immunol., 80(1)*:141-148 (1990), reported that pretreatment of mouse macrophages with 50 to 100 ug/ml LAM blocked macrophage activation by IFN-γ, but pretreatment with 10 µg/ml LAM did not affect macrophage activation. Thus, it is believed that low concentrations of LAM stimulate cytokine production, at least initially. However, higher concentrations of LAM (50-100 µg/ml or more) appear to block rather than promote macrophage function. Thus, the production of either too much or too little cytokine at different stages of *Mycobacterial* disease may contribute to *Mycobacterial* pathogenesis. New methods for blocking the above-characterized physiological effects of LAM molecules are a highly desirable goal in the treatment of subjects that are or that have been infected with *Mycobacteria*. For the same reasons, new methods by which fluids containing LAM can be decontaminated prior to administration into a subject are also desirable. Neutralization of even small amounts of LAM is desirable, because small amounts of LAM may have the physiological effect of stimulating cytokine production.

Of interest to the background of the invention are the disclosures of PCT/US88/00510, (WO 88/06038) published August 25, 1988, indicating that certain poloxypropylene/polyoxyethylene nonionic surface-active block copolymers can be used with or without conventional antibiotics to treat infection with *Mycobacterium*. This reference cites studies suggesting that the effects of other nonionic surfactants on tuberculosis are most likely due to modification of surface lipids of *Mycobacteria*, and not to direct bactericidal effects on *Mycobacteria*. See e.g. Cornforth *et al, Nature, 168*:150-153 (1951).

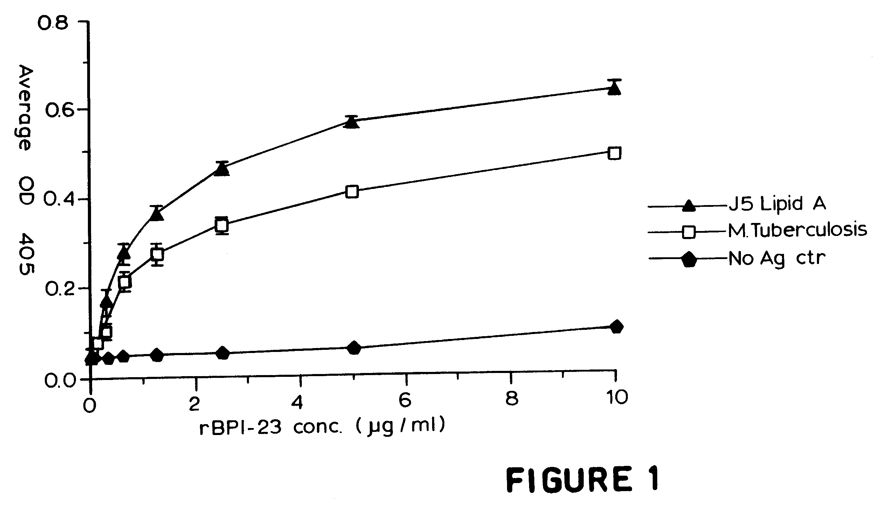

Bactericidal/permeability-increasing protein (BPI) is a protein isolated from the granules of mammalian polymorphonuclear neutrophils (PMN), which are blood cells essential in the defense against invading microorganisms. Human BPI protein has been isolated from PMN's by acid extraction combined with either ion exchange chromatography Elsbach, *J. Biol. Chem., 254*:11000 (1979) or *E. coli* affinity chromatography, Weiss, et al., *Blood, 69*: 652 (1987), and has potent bactericidal activity against a broad spectrum of Gram-negative bacteria. The molecular weight of human BPI is approximately 55,000 Daltons (55 kD). The amino acid sequence of the entire human BPI protein, as well as the DNA encoding the protein, have been elucidated in Figure 1 of Gray, *et al., J. Biol. Chem., 264*: 9505 (1989), incorporated herein by reference.

BPI has been shown to be a potent bactericidal agent active against a broad range of Gram-negative bacterial species. The cytotoxic effect of BPI has been shown to be highly specific to sensitive Gram-negative species, while non-toxic for other non-acid fast, Gram-positive bacteria and for eukaryotic cells. The precise mechanism by which BPI kills bacteria is as yet unknown, but it is known that BPI must first attach to the surface of susceptible Gram-negative bacteria. It is thought that this initial binding of BPI to the bacteria involves electrostatic interactions between the basic BPI protein and negatively charged sites on lipopolysaccharides (LPS). LPS has been referred to as endotoxin because of the potent inflammatory response that it stimulates. LPS induces the release of mediators by host inflammatory cells which may ultimately result in irreversible endotoxic shock. BPI binds to Lipid A, the most toxic and most biologically active component of LPS.

In susceptible bacteria, it is thought that BPI binding disrupts LPS structure, leads to an activation of bacterial enzymes that degrade phospholipids and peptidoglycans, alters the permeability of the cell's outer membrane, and ultimately causes cell death by an as yet unknown mechanism. BPI is also capable of neutralizing the endotoxic properties of LPS to which it binds. Because of its Gram-negative bactericidal properties and its ability to neutralize LPS, BPI can be utilized for the treatment of mammals suffering from diseases caused by Gram-negative bacteria, such as bacteremia or sepsis.

An approximately 25 kD proteolytic fragment corresponding to the amino-terminal portion of human BPI holoprotein possesses the antibacterial efficacy of the naturally-derived 55 kD human holoprotein. In contrast to the amino-terminal portion the carboxy-terminal region of the isolated human BPI protein displays only slightly detectable anti-bacterial activity. Ooi, *et al., J. Exp. Med., 174*:649 (1991). A BPI amino-terminal fragment, comprising approximately the first 199 amino acid residues of the human BPI holoprotein, has been produced by recombinant means as a 23 kD protein referred to as "rBPI₂₃". Gazzano-Santoro *et al., Infect. Immun. 60*: 4754-4761 (1992).

Of interest to the present invention is the disclosure of Kelly *et al*. at the Annual Meeting of the Society of University Surgeons, Feb 10-14, 1993, (paper #1) that intravenously administered BPI is useful for providing improved pulmonary clearance in a Gram-negative pneumonia model.

While BPI protein products are effective for treatment of conditions associated with Gram-negative bacterial infection, there continues to exist a need in the art for products for treatment of Gram-positive bacterial infections such as infection with *Mycobocteria*.

### SUMMARY OF THE INVENTION

The present invention provides use of a BPI protein product for the manufacture of a medicament for treatment of *Mycobacteria* infection. Medicaments manufactured according to the invention may be administered orally, systemically (such as by intravenous, intramuscular or other injection), or as an aerosol. *Mycobocterial* disease states according to the invention include tuberculosis, which can be caused by infection with *M. tuberculosis*, leprosy, which can be caused by infection with *M. leprae*, and diseases caused by *M. avium* and other *Mycobacteria* species. According to preferred medicaments, antibiotics and/or surfactants may be in combination with the BPI protein product.

According to another aspect of the present invention, there is provided use of a BPI protein product for the manufacture of a medicament for the treatment of the adverse physiological effects of the presence of *Mycobacterial* LAM in circulation, for example, for neutralizing the effect of low concentrations of *Mycobacterial* LAM capable of stimulating cytokine production in a host, and for neutralizing the inhibitory effect that higher concentrations of *Mycobacterial* LAM (i.e. 100µg/ml or more) have upon the interferon-mediated activation of macrophages. Specifically, a BPI protein product medicament may be used for treatment of an immunosuppressed subject failing to respond to microbes or tumor cells due to *Mycobacterial* LAM-induced insensitivity of macrophages to activation by T-cell lymphokines.

A BPI protein product may be employed in methods for decontaminating a fluid containing LAM prior to administration of the fluid into a subject. Such decontamination methods involve contacting the fluid with the BPI protein product prior to administration, under conditions such that LAM forms a complex with the BPI protein product which can be removed from the fluid. Thus, according to a further aspect of the present invention, there is provided an *in vitro* method for decontaminating a fluid containing lipoarabinomannan, said method comprising contacting the fluid with a bactericidal/permeability-increasing protein product under conditions such that lipoarabinomannan therein binds the bactericidal/permeability-increasing protein product and separating said bound materials from said fluid. Fluids subject to decontamination by the methods of this invention include, but are not limited to, blood, plasma, blood serum, bone marrow, isotonic solutions, pharmaceutical agents, and cell culture agents.

As used herein, "BPI protein product" includes naturally and recombinantly produced bactericidal/permeability-increasing protein, natural, synthetic and recombinant biologically active polypeptide fragments of bactericidal/permeability-increasing protein, and biologically active polypeptide analogs, including hybrid fusion proteins, of either bactericidal/permeability-increasing protein or biologically active fragments thereof.

### BRIEF DESCRIPTION OF THE FIGURES

Fig. 1 graphically depicts the results of an assay of BPI protein product binding to *E. coli* J5 Lipid A and *M. tuberculosis*.

### DETAILED DESCRIPTION OF THE INVENTION

The present invention is based on the discovery that a composition comprising a BPI protein product can be administered for effective treatment of a subject suffering from infection with *Mycobacteria*. In particular, the invention provides for treatment of leprosy and tuberculosis, grave diseases caused by the species *M. leprae* and *M. tuberculosis*, respectively. It is contemplated that the medicaments described herein may be used to treat infection with other *Mycobacterial* species, most notably *M. avium*, but also *M. marinum, M. fortuitum, M. chelonei*, and the like. Beneficial effects of treatment with BPI protein products are expected to result from binding of the products to LAM and to disruption of the bacterial cell wall components (with or without direct killing of the bacteria) in manner similar to that resulting from treatment of Gram-negative disease states.

The BPI protein products including biologically active fragments of BPI holoprotein which are to be used in the manufacture of a medicament according to this invention may be generated and/or isolated by any means known in the art. Co-owned, copending U.S. Patent Application Ser. No. 07/885,501, published as WO93/23540 (European Patent Application No 93913992.9), discloses novel methods for the purification of recombinant BPI protein products expressed in and secreted from genetically transformed mammalian host cells in culture and how one may produce large quantities of recombinant BPI protein products suitable for incorporation into stable, homogeneous pharmaceutical preparations.

Biologically active fragments of BPI include biologically active molecules that contain the same amino acid sequence as a BPI holoprotein, except that the molecules lack amino-terminal amino acids, internal amino acids, and/or carboxy-terminal amino acids of the holoprotein. By way of nonlimiting examples, such fragments include the previously mentioned natural 25 kD fragment and the recombinant 23 kD, 199 amino acid residue amino-terminal fragment of human BPI holoprotein referred to as rBPI₂₃. See, Gazzano-Santoro *et al., Infect. Immun. 60*:4754-4761 (1992). In that publication, an expression vector was used as a source of DNA encoding a recombinant expression product (rBPI₂₃) having the 31-residue signal sequence and the first 199 amino acids of the amino-terminus of the mature human BPI, as set out in SEQ ID NOs: 1 and 2 taken from Gray *et al., supra*, except that valine at position 141 is specified by GTG rather than GTC and residue 185 is glutamic acid (specified by GAG) rather than lysine (specified by AAG).

Biologically active fragments of BPI also include, but are not limited to those polypeptides disclosed in co-owned, co-pending, U.S. Patent Application Serial No. 08/030,644, filed March 12, 1993 by Little *et al*., entitled "Therapeutic Uses of Bactericidal/Permeability-Increasing Protein Products" and noted to display the LPS binding, bactericidal and heparin binding activities of BPI holoprotein, published as WO94/20128 (European Patent Application No 94910854.2).

Biologically active polypeptide analogs of BPI include but are not limited to biologically active hybrid fusion proteins comprising a BPI protein or biologically active fragment thereof, and at least a portion of at least one other polypeptide. Such proteins are described by Theofan *et al*. in co-owned, co-pending U.S. Pat. App. Ser. No. 07/885,911, published as WO93/23434, and include hybrid fusion proteins comprising, at the amino terminal end, a BPI protein or a biologically active fragment thereof and, at the carboxy terminal end, at least one constant domain of an immunoglobulin heavy chain or allelic variant thereof.

Biologically active peptide analogs of BPI also include but are not limited to a BPI protein or biologically active fragment thereof, wherein one or more amino acid residues has been replaced by a different amino acid. For example, co-owned, copending U.S. Patent Application Ser. No. 08/013,801 (Theofan *et al*., "Stable Bactericidal/Permeability-Increasing Protein Products and Pharmaceutical Compositions Containing the Same," filed February 2, 1993, published as WO94/18323 (European Patent Application No 94908704.3), discloses polypeptide analogs of BPI and of BPI fragments wherein a cysteine residue at position 132 or at position 135 is replaced by a different amino acid.

The administration of a BPI protein product is preferably accomplished with a pharmaceutical composition comprising the BPI protein product and a pharmaceutically acceptable diluent, adjuvant, or carrier. The BPI protein product composition may be administered without or in conjunction with known antibiotics, surfactants, or other chemotherapeutic agents. A preferred pharmaceutical composition comprises the BPI protein at a concentration of 1 mg/ml in citrate buffered saline (0.02 M citrate, 0.15 M NaCl, pH 5.0) comprising 0.1% by weight of poloxamer 188 (Pluronic F-68, BASF Wyandotte, Parsippany, NJ) and 0.002% by weight of polysorbate 80 (Tween 80, ICI Americas Inc., Wilmington, DE). Such preferred combinations are taught in a co-owned, copending, U.S. Patent Application Ser. No. 08/012,360 (McGregor et al., "Improved Pharmaceutical Compositions" filed February 2, 1993); published as WO94/17819 (European Patent Application No 94907963.6). The surfactants which may be employed may also have the advantage of increasing the availability to BPI protein products of LAM binding sites, by removing some of the waxy *Mycobacterial* cell wall components; surfactants may also enhance the disassociative activity of BPI protein products on the cell membrane. Some surfactants are themselves anti-*Mycobacterial* and may synergize BPI protein products and vice versa.

The BPI protein product can be administered by any known method, such as orally, systemically (such as by intravenous, intramuscular or other injection), or as an aerosol. Medicaments can be prepared for oral administration or by injection or other parenteral methods and preferably include conventional pharmaceutically acceptable carriers and adjuvents as would be known to those of skill in the art. The medicaments may be in the form of a unit dose in solid, semi-solid and liquid dosage forms such as tablets, pills, powders, liquid solutions or suspensions, and injectable and infusible solutions. Effective dosage ranges from about 100 µg/kg to about 10 mg/kg of body weight are contemplated. Intravenous administration is a preferred method for treatment of leprosy.

It is contemplated that aerosol administration to the lungs will be a preferred method for treating other *Mycobacterial* infections, such as tuberculosis. Such aerosol formulations would be manufactured by means that are known in the art, and administered by metered dose inhaler, updraft nebulization, or other means known in the art.

An aspect of the present invention is to provide the use of a BPI protein product for the manufacture of a medicament for treating a subject suffering from any of the physiological effects of *Mycobacterial* LAM. As described above, the physiological effects of LAM depend on a number of factors, including the source and concentration of the LAM, and the length of time to which host cells are exposed to LAM. Example 3, infra, demonstrates that 20 - 100 µg/ml of nonviable, desiccated *M. tuberculosis* added to whole blood will stimulate TNF production by the monocytes in the blood. Other studies described above have shown that 50-100 µg/ml of LAM will down-regulate macrophage functions and expression (TNF, and the like) and prevent macrophage activation, said methods comprise administering a BPI protein product to the subject. Medicaments are provided for treating a subject suffering from the effects of increased cytokine production caused by the physiological presence of LAM. Medicaments are also provided for treating a subject suffering from LAM-induced inhibition of macrophage activation, and the effects thereof. Medicaments and formulations by which a BPI protein product may be administered, including preferred medicaments and formulations, are the same as those set forth above for the treatment of *Mycobacterial* infection.

Because of the harmful physiological effects that *Mycobacterial* LAM can have on a subject, even in the absence of viable *Mycobacteria*, methods are provided in the present invention by which a fluid containing LAM may be decontaminated in vitro, prior to administration of the fluid into a subject. Such in vitro methods comprise contacting the fluid with a BPI protein product prior to administration, under conditions such that LAM forms a complex with the BPI protein product, thereby decontaminating the fluid. By way of nonlimiting examples, such methods may be applied to fluids such as blood, plasma, blood serum, bone marrow, isotonic solutions, pharmaceutical agents, or cell culture reagents.

This invention provides the use of a composition comprising a BPI protein product for the manufacture of a medicament for the therapeutic application of treating any of the aforementioned conditions or diseases from which a subject suffers. The medicament may include, in addition to a BPI protein product, other chemotherapeutic agents such as antibiotics or surfactants. The medicament may additionally or alternatively include one or more additional pharmaceutically acceptable components, such as diluents, adjuvants, or carriers.

Example 1 address BPI protein products binding to a species of *Mycobocterium, M. tuberculosis*. Example 2 address prospective use of BPI protein products in binding purified LAM of *Mycobacteria*. Example 3 describes attempts to reverse *Mycobacteria*-induced cytokine production in whole human blood. Remaining Examples 4-11 address prospective *in vitro* and *in vivo* use of BPI protein products according to this invention. The models described in those examples and/or other models known in the art are used to predict the efficacy and the optimal BPI protein product formulations of the methods of invention.

### EXAMPLE 1

An enzyme linked immunosorbent assay (ELISA) was conducted to determine binding of a BPI protein product to *M. tuberculosis*. Specifically, non-viable, desiccated *M. tuberculosis* H37 RA (Difco, Detroit MI) was suspended in DPBS (25 µg/ml) and used to coat microtiter wells overnight at 37°C. Wells were also coated with either 25 µg/ml Lipid A (*E.coli J5* mutant, RIBI, Hamilton MT) or 500 µl DPBS to demonstrate the functionality and specificity of rBPI₂₃. After washing (3x with DPBS + 0.05% Tween 20), the plates were blocked for 1hr. at room temperature with 200 µl/well of DPBS + 1% non-fat milk. After washing as above, 50 µl solutions of either various concentrations of rBPI₂₃ (in DPBS containing 0.05% Tween 20) or DPBS (negative control) were added to the wells, which were then incubated for 1 hr. at 37°C. The wells were again washed as above, and the amount of rBPI₂₃ bound to the wells was determined using an anti-rBPI₂₃ mouse monoclonal antibody (designated αBPI MAb-2-4) and an enzyme conjugated anti-murine IgG antibody (HRP-Ab, Zymed #61-0120, San Francisco, CA). To each well 100 µl of αBPI MAb-2-4 was added (100 ng/ml in DPBS + 0.05% Tween 20), and the plates were incubated 1 hr. at 37°C. After washing as above, 100 µl of HRP-Ab was added (1:1000 in DPBS + 0.05% Tween 20) to each well and the plates were again incubated 1 hr. at 37°C. After washing the plates as above, 100µl substrate in 0.1M citrate plus 1:50 ADS (20 mg/ml stock) and 1:1000 H₂O₂ was added to each well. The plates were incubated 10-30 min. at room temperature, and absorbance readings were taken at 405 nm (OD 405).

The results of the experiment are represented graphically in Figure 1, which depicts the ability of varying concentrations rBPI₂₃ to bind to J5 Lipid A (filled triangles); to M. tuberculosis (open squares); and to the no antigen-free control (filled circles). The abscissa of each measurement represents the concentration of rBPI₂₃, and the ordinate represents the average OD 405 measurements from four trials. Error bars reflect the variation in OD 405 readings for each data point.

This experiment demonstrated that rBPI₂₃ binds specifically to non-viable desiccated *M.tuberculosis*. The functionality of the rBPI₂₃ used in these experiments was confirmed by the results of the Lipid A (positive control) binding assay, and the specificity of the experiments was confirmed by the lack of binding to the negative control samples.

### EXAMPLE 2

In this example, an ELISA Assay is conducted to determine binding of a BPI protein product to the lipoarabinomannan portion of *Mycobacteria*. The binding activity of BPI protein product (e.g., rBPI₂₃) to LAM is demonstrated as described in the previous example, except LAM purified from a species of *Mycobacterium*, (e.g., *M.tuberculosis* or *M.leprae*) is substituted for the nonviable *M.tuberculosis* used to coat the ELISA plates in that example. Purified LAM is isolated as described by Hunter *et al., J. Biol. Chem., 261*:12345-12351 (1986). Specific binding of biologically active BPI protein product is demonstrated by comparison of the OD 405 readings from the LAM coated wells with positive and negative controls.

### EXAMPLE 3

The following experiment was conducted to determine the effect of a BPI protein product, rBPI₂₃, on *Mycobacteria*-induced cytokine production in whole human blood. Whole human blood from healthy volunteers was collected into Vacutainer tubes (ACD, Beckton Dickinson, Rutherford, NJ). Aliquots of blood (225 µl) were mixed with either rBPI₂₃ (10 µg/ml final) or the protein thaumatin (10 µg/ml final in 5 ml) as a negative control. RPMI medium (20 µl) was added to each sample. Varying dilutions (0 - 8 ng/ml) of either *E. coli* O113 LPS (Ribi, Hamilton MT) or of non-viable, desiccated *M. tuberculosis* H37 RA (0 - 100 µg/ml) (Difco, Detroit MI) were added to the samples, which were then incubated at 37°C for 6 hours. The reactions were stopped by the addition of 750 µl of RPMI medium, the samples were centrifuged at 500 g for 7 min, and stored at -20°C until analyzed. The supernatant was assayed for cytokine (TNF) levels based on a standard curve, according to the manufacturers' recommendation (Biokine ELISA test, T Cell Sciences, Cambridge, MA).

The assay results revealed that rBPI₂₃ at 10 µg/ml had no inhibitory effect on *M. tuerculosis*-induced TNF release at the concentration (20 - 100 µg/ml) of *M. tuberculosis* added to the blood samples. The same concentration of rBPI₂₃ eliminated LPS-induced TNF release at the LPS concentrations tested (2 - 8 ng/ml). The lack of inhibitory effect on cytokine induction by *M. tuberculosis* may be the result of use of sub-optimal dosage levels. Alternatively, some component of the *Mycobacterial* cell wall other than the LAM bound by rBPI₂₃ may be responsible for inducing cytokine production at the *Mycobacterium* concentrations tested.

### EXAMPLE 4

The following experiment is conducted to determine the *in vitro* inhibitory effect of a BPI protein product on the growth of a *Mycobacterium* species, *Mycobacterium tuberculosis* (MTB). The procedure can be performed with other cultivable *Mycobacterial* species and employs concentrations of a BPI protein product that would be readily generated in human serum by ordinary modes of oral or parenteral administration and/or readily delivered to lung surface by aerosol administration. The effects of the BPI protein product can be evaluated with and without non-ionic surfactants, and/or standard antibiotics.

Log phase cultures of antibiotic-sensitive and antibiotic-resistant MTB are incubated in either 7H11 broth medium or whole human blood, to which the following is added: (a) nothing; (b) surfactant; (c) standard MTB antibiotic; (d) antibiotic plus surfactant. Cultures are incubated with varying concentrations of, e.g., rBPI₂₃. Duplicate cultures grown in each medium are also left untreated by rBPI₂₃ as a negative control. The organisms are placed in Bactec® bottles (Johnston Laboratories, Cockeysville, MD) containing ¹⁴C labeled nutrients. RBPI₂₃ challenged *M. tuberculosis* growth is determined by measuring the elution of ¹⁴CO₂ from the medium, compared to the appropriate negative control. The absence of the formation ¹⁴CO₂ by the treated cultures is indicative of the inhibitory affects of rBPI₂₃ to MTB. Differential amounts of ¹⁴CO₂ formed in the absence or presence of standard MTB antibiotics and/or surfactants is indicative of the synergistic or additive effect that a BPI protein product has when used conjunctively with such agents. By comparing the results of this experiment performed with varying concentrations of the BPI protein product, the effective concentration of the BPI protein product is optimized. Radiometric assays to test the susceptibility of *Mycobacterial* species to drugs have been described previously. *See* McClatchy (cited *supra*) and references therein.

### EXAMPLE 5

The following experiment is conducted to determine the *in vitro* effects of a BPI protein product (rBPI₂₃) in an *M. leprae* model. A palmitic acid oxidation assay is used to measure the "viability" of the uncultivable leprosy bacillus adhered to filter paper and "grown" in a ¹⁴C-palmitic acid-containing medium. In this method ¹⁴CO₂ evolved from the metabolism by *M.leprae* of ¹⁴C-palmitic acid is traps on filter paper moistened with NaOH and radioactivity is determined with a liquid scintillation counter. Susceptibility to BPI protein product formulations is determined by differences in radioactivity for *M.leprae* tested with such formulations and treated control cultures.

### EXAMPLE 6

The following experiment, which is a variation of an assay conducted by Mittal *et al., J. Clin. Microbiol*., *17(4)*:704-707 (1983), is conducted to determine the *in vitro* inhibitory effect of BPI protein product on the growth of *Mycobacterium leprae*. The effects of different concentrations of BPI protein product on *M. leprae* are evaluated with and without non-ionic surfactants, and/or standard antibiotics. The procedures as described by Mittal *et al*. are outlined below.

Skin biopsy specimens from lepromatous patients are homogenized and are used to inoculate suspensions of mouse peritoneal macrophages cultured in RPM 1640 (GIBCO Biocult, Irvine, Scotland) enriched with 30% fetal calf serum. After incubating 18 hours, fresh media containing [*methyl*-³H]-thymidine (Amersham International Ltd., Arlington Heights, IL) is added and the cultures are incubated for 14 days. The procedure of Mittal *et al*. is varied by testing the effect of different concentrations of BPI protein product with or without surfactants and/or antibiotics on ³H-thymidine incorporation. Macrophages containing phagocytosed viable *M. leprae* will incorporate ³H-thymidine at a 2 to 10-fold higher rate than control cultures containing heat killed *M. leprae*. Greater than 50% inhibition of ³H-thymidine-incorporation is indicative of bactericidal efficacy of the test product.

### EXAMPLE 7

An experiment is conducted to determine the *in vivo* effect that a BPI protein product will have on *M. tuberculosis* species. The model employed is a variation of that used by Lalande *et al., Antimicrobial Agents and Chemotherapy, 37(3)*:407-413 (1993), to assess the efficacy of antimicrobial agents against *M.tuberculosis*. Mice inoculated intravenously with *M.tuberculosis* are treated with various BPI protein product doses alone or in combination with surfactants and/or antibiotics. The efficacy of such treatment regiments is analyzed as described.

### EXAMPLE 8

The following experiment is conducted to determine the effect that a BPI protein product will have on *M. leprae in vivo*. The model to be used is a variation of that developed by Shepard to study the effect of compounds on the growth of *M. leprae* in the footpads of infected mice. Shepard *et al., Proc. Soc. Exp. Biol. Med., 109*:636-638 (1962); Shepard, *J. Exp. Med. 112*:445-454 (1960). Briefly, leprosy bacilli are inoculated into foot-pads of mice, which are subsequently treated with different amounts of test compound with or without known antibiotics and/or surfactants. Untreated infected mice are used as a control. Mice from each treatment regimen are sacrificed at monthly intervals, and sections cut from the infected foot. The presence of an area containing acid-fast bacteria can be observed microscopically and/or the number of such bacteria can be counted. See Shepard and McRae, *Int. J. Lepr., 36*:78-82. Differences between *M. leprae* bacteria levels observed in treated versus control mice is indicative of the bacteriostatic or bactericidal efficacy of a given BPI treatment regimen. The metabolic status of isolated *M. leprae* may also be measured. Franzblau and Hastings, *Antimicrobial Agnes and Chemotherapy, 31(5)*:780-783 (1987).

### EXAMPLE 9

The following experiments are designed to demonstrate that BPI protein product is able to inhibit the ability of low concentration of LAM to induce cytokines, yet reverse the unresponsive state that attends higher concentrations of LAM. Increasing concentrations of LAM are pretreated with BPI protein product at varying concentrations. These complexes are applied to peritoneal macrophages from normal and *Mycobacterium* species infected mice. TNF production by treated cells will be assessed.

### EXAMPLE 10

A variation of the armadillo model developed by Kirchheimer *et al., Int. J. Lepr., 39*:693-702 (1971); *Id., 40*:229 (1972), is employed to study the *in vivo* effect of BPI protein product test compositions on the growth of *M. leprae* in infected armadillos. Briefly, leprosy bacilli are inoculated into armadillos, which are subsequently treated with different amounts of a test composition. The test compositions will comprise a BPI protein product, e.g. rBPI₂₃, with or without known antibiotics and/or surfactants. Untreated infected specimens are used as a control. Armadillos from each treatment regimen are examined and biopsy specimens analyzed by procedures known in the art. *M. leprae* isolated from armadillos is assayed for metabolic activity. Differences between the appearance of lesions, differences in *M. leprae* bacterial concentrations, and differences in the metabolic activity of *M. leprae* isolates in treated versus control specimens are indicative of the bacteriostatic or bactericidal efficacy of a given BPI treatment regimen.

### EXAMPLE 11

The following experiment is conducted to determine the level of decontamination of a fluid containing LAM that can be achieved by treatment with a BPI protein product. Whole human blood, plasma, blood serum or the like is passed through a column containing a matrix, to which a BPI protein product is bound. Such matrix may be constructed by any means known to those skilled in the art. LAM in the fluid complexes with the BPI protein product affixed to the matrix as the fluid is passed through the column. The absence of LAM in the fluid eluted from the column demonstrates the effectiveness of a BPI protein product at decontaminating a fluid containing LAM.

Alternatively, monoclonal antibodies with binding specificity for a BPI protein product, such as the antibodies employed in Example 1, are affixed to the matrix. A sufficient amount of a BPI protein product is added to the mixture to bind any LAM present in the fluid. The fluid is purified by passing it through the column. The αBPI antibodies affixed to the column bind the LAM/BPI protein product complex in the fluid, and the fluid eluted from the column is analyzed for the presence or absence of LAM contamination.

## Claims

1. Use of a bactericidal/permeability-increasing protein product for the manufacture of a medicament for treatment of *Mycobacteria* infection.

2. The use of Claim 1, wherein the medicament is to be administered orally.

3. The use of Claim 1, wherein the medicament is to be administered intravenously.

4. The use of Claim 1, wherein the medicament is to be administered as an aerosol.

5. The use of any one of the preceding claims, wherein the bactericidal/permeability-increasing protein product is a 23-25 kD amino-terminal fragment of bactericidal/permeability-increasing holoprotein.

6. The use of any one of the preceding claims, for the treatment of infection with a *Mycobacterium* species bacterium selected from *M.tuberculosis, M.leprae* and *M.avium*.

7. The use of any one of the preceding claims, wherein the medicament further comprises an antibiotic.

8. The use of any one of the preceding claims, wherein the medicament further comprises a surfactant.

9. The use of any one of the preceding claims for treatment of the adverse physiological effects of the presence of *Mycobacterial* lipoarabinomannan in circulation.

10. The use of Claim 9, wherein the adverse physiological effects comprise compromised immune response to microbes or tumor cells due t*o Mycobcaterial* lipoarabinomannan-induced inhibition of macrophage activation by T-cell lymphokines.

11. The use of Claim 9, wherein the adverse physiological effects comprise increased production of a cytokine by the subject.

12. An *in vitro* method for decontaminating a fluid containing lipoarabinomannan, said method comprising contacting the fluid with a bactericidal/permeability-increasing protein product under conditions such that lipoarabinomannan therein binds the bactericidal/permeability-increasing protein product and separating said bound materials from said fluid.

13. The method of Claim 12, wherein the fluid is selected from blood, plasma, blood serum, and bone marrow.

14. The method of Claim 12, wherein the fluid is selected from an isotonic solution, a pharmaceutical agent, and a cell culture reagent.

## Patentansprüche

1. Verwendung eines bakterientötenden/durchlässigkeitserhöhenden Proteinproduktes zur Herstellung eines Arzneimittels zur Behandlung von Mycobacteria-Infektion.

2. Die Verwendung nach Anspruch 1, wobei das Arzneimittel oral verabreicht werden soll.

3. Die Verwendung nach Anspruch 1, wobei das Medikament intravenös verabreicht werden soll.

4. Die Verwendung nach Anspruch 1, wobei das Medikament als ein Aerosol verabreicht werden soll.

5. Die Verwendung nach einem der vorangehenden Ansprüche, wobei das bakterientötende/durchlässigkeitserhöhende Proteinprodukt ein 23-25 kD aminoterminales Fragment eines bakterientötenden/durchlässigkeitserhöhenden Holoprotein ist.

6. Die Verwendung nach einem der vorangehenden Ansprüche, zur Behandlung einer Infektion mit einem Bakterium der Mycobacterium-Spezies, ausgewählt aus M. tuberculosis, M. leprae und M. avium.

7. Die Verwendung nach einem der vorangehenden Ansprüche, wobei das Arzneimittel außerdem ein Antibiotikum umfaßt.

8. Die Verwendung nach einem der vorangehenden Ansprüche, wobei das Arzneimittel außerdem eine oberflächenaktive Substanz umfaßt.

9. Die Verwendung nach einem der vorangehenden Ansprüche zur Behandlung der nachteiligen physiologischen Wirkungen des Vorhandenseins von mycobakteriellem Lipoarabinomannan im Kreislauf.

10. Die Verwendung nach Anspruch 9, wobei die nachteiligen physiologischen Wirkungen die beeinträchtigte Immunreaktion auf Mikroben oder Tumorzellen aufgrund einer durch mycobakterielles Lipoarabinomannan induzierten Inhibition der Makrophagenaktivierung durch T-Zell-Lymphokine umfaßt.

11. Die Verwendung nach Anspruch 9, wobei die nachteiligen physiologischen Wirkungen die erhöhte Produktion eines Zytokins durch den Patienten umfaßt.

12. Ein in-vitro-Verfahren zur Dekontaminierung einer Flüssigkeit, die Lipoarabinomannan enthält, wobei besagtes Verfahren umfaßt, daß die Flüssigkeit mit einem bakterientötenden/durchlässigkeitserhohenden Proteinprodukt unter derartigen Bedingungen in Kontakt gebracht wird, daß Lipoarabinomannan darin das bakterientotende/durchlässigkeitserhöhende Proteinprodukt bindet, und daß besagte gebundene Materialien aus besagter Flüssigkeit abgetrennt werden.

13. Das Verfahren nach Anspruch 12, wobei die Flüssigkeit ausgewählt wird aus Blut, Plasma, Blutserum und Knochenmark.

14. Das Verfahren nach Anspruch 12, wobei die Flüssigkeit ausgewählt wird aus einer isotonischen Lösung, einem pharmazeutischen Mittel und einem Zellkulturreagens.

## Revendications

1. Utilisation d'un produit protéique bactéricide/accroissant la perméabilité pour la production d'un médicament pour le traitement d'une infection à *Mycobacteria*.

2. Utilisation selon la revendication 1, dans laquelle le médicament est administré par voie orale.

3. Utilisation selon la revendication 1, dans laquelle le médicament est administré par voie intraveineuse.

4. Utilisation selon la revendication 1, dans laquelle le médicament est administré sous forme d'aérosol.

5. Utilisation selon l'une quelconque des revendications précédentes, dans laquelle le produit protéique, bactéricide/accroissant la perméabilité, est un fragment amino-terminal de 23-25 kD.

6. Utilisation de l'une quelconque des revendications précédentes pour le traitement de l'infection par une des espèces de bactéries *Mycobacterium* choisies parmi *M.tuberculosis, M.leprae* et *M.avium*.

7. Utilisation selon l'une quelconque des revendications précédentes, dans laquelle le médicament comprend en outre un antibiotique.

8. Utilisation de l'une quelconque des revendications précédentes, dans laquelle le médicament comprend en outre un agent tensio-actif.

9. Utilisation selon l'une quelconque des revendications précédentes pour le traitement des effets physiologiques négatifs de la présence de lipoarabinomannane mycobactérien en circulation.

10. Utilisation de la revendication 9, dans laquelle les effets physiologiques négatifs comprennent une réponse immunitaire compromise à des microbes ou à des cellules tumorales due à l'inhibition, induite par le lipoarabinomannane mycobactérien, de l'activation des macrophages par les lymphokines des cellules T.

11. Utilisation de la revendication 9 dans laquelle les effets physiologiques négatifs comprennent l'accroissement de la production d'une cytokine par un sujet.

12. Procédé *in vitro* pour décontaminer un fluide contenant du lipoarabinomannane, ledit procédé comprenant la mise en contact du fluide avec un produit protéique bactéricide/augmentant la perméabilité dans des conditions telles que le lipoarabinomannane qui s'y trouve lie le produit bactéricide/augmentant la perméabilité, et la séparation desdites matières liées d'avec ledit fluide.

13. Procédé selon la revendication 12, dans lequel le fluide est choisi parmi le sang, le plasma, le sérum sanguin, et la moëlle osseuse.

14. Procédé selon la revendication 12, dans lequel le fluide est choisi parmi une solution isotonique, un agent pharmaceutique et un réactif de culture cellulaire.
